(19) Europäisches Patentamt
European Patent Office
Office européen des brevets

(11) EP 2 145 895 B1

(12) **EUROPEAN PATENT SPECIFICATION**

(45) Date of publication and mention of the grant of the patent:
**30.10.2013 Bulletin 2013/44**

(51) Int Cl.:
***C07H 1/00*** *(2006.01)* ***B01J 19/00*** *(2006.01)*

(21) Application number: **08290671.0**

(22) Date of filing: **08.07.2008**

(54) **Process for the manufacturing of glycochips**

Verfahren zur Herstellung von Glycochips

Processus de fabrication de glycochips

(84) Designated Contracting States:
**AT BE BG CH CY CZ DE DK EE ES FI FR GB GR HR HU IE IS IT LI LT LU LV MC MT NL NO PL PT RO SE SI SK TR**

(43) Date of publication of application:
**20.01.2010 Bulletin 2010/03**

(73) Proprietors:
- **Commissariat à l'Énergie Atomique et aux Énergies Alternatives**
  **75015 Paris (FR)**
- **Azbil Corporation**
  **Chiyoda-ku**
  **Tokyo (JP)**

(72) Inventors:
- **Robert, Véronique**
  **38000 Grenoble (FR)**
- **Hoang, Antoine**
  **38000 Grenoble (FR)**
- **Yamasaki, Shinsuke**
  **Yokohama-shi 224-0037 (JP)**

(74) Representative: **Orès, Bernard et al**
**Cabinet ORES**
**36, rue de St Pétersbourg**
**75008 Paris (FR)**

(56) References cited:
**WO-A-00/53310** **WO-A-01/40796**

- **HOUSEMAN B T ET AL: "Maleimide-functionalized self-assembled monolayers for the preparation of peptide and carbohydrate biochips" LANGMUIR, ACS, WASHINGTON, DC, US, vol. 19, no. 5, 4 March 2003 (2003-03-04), pages 1522-1531, XP002349565 ISSN: 0743-7463**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 145 895 B1

## Description

The present invention relates to a process for the manufacturing of solid supports functionalized by saccharide type molecules (glycochips or carbohydrate arrays or alternatively oligosaccharide arrays). The present invention also relates to the glycochips directly obtained by such a manufacturing process and to their use, in particular for biological analysis and especially for the screening of saccharides or proteins such as Hepatocyte Growth Factors (HGFs) or for the study of saccharides/proteins interactions.

The development of DNA chip technologies has made possible a significant advance in programs related to functional genomics. This is because the miniaturization of techniques for the deposition or synthesis of DNA has resulted in DNA analyses being carried out in parallel, and thus according to multiple parameters, on chips. More recently, the emergence of proteomics has given rise to the concept of protein chips. The latter make possible the analysis in parallel of interactions of protein/ligand type.

More recently still, biological research has taken an interest in "glycomics", that is to say in the systematic study of carbohydrate/protein interactions. This is because glycoconjugates (that is to say, any molecule having a domain of glycan type, such as glycoproteins, glycolipids, proteoglycans, glycoaminoglycans and more generally any molecule comprising carbohydrates) have a particularly broad functional repertoire. Chemically, these carbohydrates are molecules constructed by the assembling of simple monomeric blocks. These assemblages can be of natural origin, and optionally fractionated, or of synthetic origin. The various functions of the molecules belonging to the family of the carbohydrates is based on the ability of the carbohydrate structures to interact with a very large number of molecules. The analysis of the mechanisms of recognition between carbohydrates and other molecules is a rapidly developing field of research. It should in particular make it possible to result in the design of novel therapeutic molecules and in a better appreciation of the toxicological risks of certain molecules. Currently, there exist few systematic methods which make it possible to produce saccharide molecules. For this reason, the determination of the structural characteristics involved in an interaction between a molecule and a carbohydrate and the characterization of the interaction itself imply the undertaking of lengthy and tedious studies.

It is therefore necessary, to make progress in the knowledge of the mechanisms of interaction between the molecules of saccharide type and their ligands, to be able to screen libraries of molecules of saccharide type with regard to a specific ligand, for example.

This is why it is found today that a novel type of biochip is emerging: various types of glycochip or carbohydrate array or alternatively oligosaccharide array, which constitute a development of the DNA or protein chip concerned with above, have thus been provided by various authors.

These glycochips are either the result of a deposition on a given substrate of a natural or synthetic saccharide substance (*ex situ* synthesis) or the result of a supported multiparallel synthesis (combinatorial chemistry) of various oligosaccharide sequences (*in situ* synthesis) representative of the molecular diversity of certain large families of endogenous glucoconjugates, such as heparans, for example.

The invention which will be described below is part of this latter technology (*in situ* synthesis).

For *in situ* synthesis, whatever the nature of the chip (ADN, proteins, saccharides), different ways of addressing the binding sites have already been used:

- Manual addressing : US patent No 5,474,796, for example, proposes a manual addressing with a microrobot according to which, functionalized sites are formed on a support surface by using photoresist substances or masks to define the different binding sites of the substrate before forming the oligonucleotide sequences by injecting the corresponding reactives (oligonucleotides) with a piezoelectric pump.
- Lithographic techniques such as disclosed in US patent No 5,658,734 which describes a process for synthesizing on a single substrate a plurality of chemical compounds having diverse structure, such a process involving the use of a bilayer photoresist to build up selected regions of the array in a step wise fashion. According to this process, the following steps are carried out: i) the deposit of a coating layer of protective polymer onto a layer of first molecules which are disposed on a substrate and have a labile protective group, ii) the deposit of a coating layer of radiation sensitive resist onto the layer of protective material, iii) imagewise exposing the resist layer to radiation, iv) developing the image to imagewise expose a portion of the layer of first molecules, v) treating the exposed portion of the layer of first molecules to remove the protecting group, and vi) bonding second molecules to the exposed first molecules.
- Photolithographic techniques: as an example, International Application WO 97/39151 describes a method for the preparation of arrays of polymer sequences wherein each array includes a plurality of different, positionally distinct polymer sequences having known monomer sequences in which the surface of the substrate is first functionalized with photolabile protected functional groups before being exposed to light radiations trough a mask to remove the protecting groups to activate the functional groups that are then coupled with a chemical monomer. The activation and coupling sequence at each region on the substrate determines the sequence of the polymer synthesized thereon. The process is particularly suited for the preparation of nucleic acid chips.

- Chemical masking: as described for example in EP-A-0 728 520 which discloses a method to form an array of polymers, such as oligonucleotides and related polymers (e.g.; peptides nucleic acids) at selected regions of a substrate using conventional linkage chemistries and which includes use of selected printing techniques in distributing materials such as barrier materials to selected regions of a substrate, said barrier material being applied as a liquid or a vapour by a variety of techniques including brush, spray techniques, printing techniques, and others.
- Mechanical addressing such as according to the method disclosed for example in EP-A-1 163 049 which describes a process for producing a matrix of sequences of chemical or biological molecules on a substrate in the form of a microplate having a plurality of microcuvettes comprising the locally depositing of a protective polymer onto functionalized microcuvettes to form solid polymer caps on all microcuvettes to allow a subsequent step of passivation of the surface surrounding each microcuvette by a non-photolabile protective group before eliminating the protection polymer from all the microcuvettes.

However, all the above-mentioned methods are often long, complicated, and necessitate the use of specific onerous reactives and materials. They also often lead to devices which do not allow immobilization of the biological molecules of interest in a sufficient sensitive manner because of a too high level of nonspecific absorption of molecules other than the molecules of interest whose immobilization is desired. The sensitivity of a functionalized solid support depends on the amount of immobilization and on the method for detecting a signal, but also and especially on the background noise level (nonspecific signal). A decrease in background noise improves the signal/noise ratio. In fact, in a device in which the presence of biological species is detected close to the surface, the background noise comes essentially from the nonspecific absorption of molecules other than the biological molecules of interest whose immobilization is desired, and which must be limited.

Therefore, to date, it has not been possible to obtain, in a completely satisfactory manner, solid supports functionalized by saccharide type molecules which allow the immobilization of proteins of interest in a reproducible and sensitive manner, and the detection of a signal by limiting the signal/noise ratio.

For these reasons, the inventors have given themselves the aim of producing such supports and have developed what forms the subject of the present invention.

A first subject matter of the present invention is thus a method for the *in situ* synthesis of saccharide type molecules onto the surface of a solid support, said surface being modified with hydroxyl functional groups, wherein said method comprises the following steps:

a) coupling, on said surface hydroxyl functional groups, a first saccharide moiety $SM_1$ having at least one hydroxyl function protected with a protecting group P, by contacting at least one area $A_1$ of said surface with a solution, in a solvent, of said first saccharide moiety $SM_1$;
b) passivating the unreacted surface hydroxyl functional groups by contacting the surface of the solid support with at least one compound of formula (I) below:

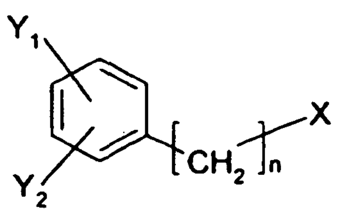

(I)

in which:

- $Y_1$ and $Y_2$, which may be identical or different, represent a hydrogen atom, a halogen atom, a $C_1$-$C_4$ alcoxy radical, a linear or branched $C_1$-$C_4$ alkyl radical, a thio($C_1$-$C_4$) alkyl radical, a nitro group, an azido group, a trifluoro($C_1$-$C_4$) alkyl radical, a cyano group or an aryl ring;
- n is an integer ranging from 1 to 4 inclusive;
- X is selected from the group consisting of a halogen atom, a trichloroacetimide group, a xanthate group -SC=SOR$_1$ in which $R_1$ represents a linear or branched $C_1$-$C_4$ alkyl radical, a thio($C_1$-$C_4$) alkyl group, a thioaryl group, a phosphate group, a phosphite group, a seleno($C_1$-C4) alkyl group, selenoaryl group, a $C_1$-$C_5$ alcoxy radical or a sulfoxide group -S(O) -$R_2$ in which $R_2$ represents linear or branched $C_1$-$C_4$ alkyl radical or an aryl ring;

with the proviso that when one of $Y_1$ and $Y_2$ represents hydrogen and the other of $Y_1$ and $Y_2$ is a methoxy radical,

then the methoxy radical is not in the para position with regards to the carbon atom bearing the $-(CH_2)_n-X$ chain;

c) reiterating coupling steps a) until the obtaining of a plurality of saccharide type molecules of determined saccharide sequences wherein each coupling steps a) is performed on at least one selected area $A_2$, $A_3$, $A_4$..., $A_m$ of the surface, said area $A_2$, $A_3$, $A_4$..., $A_m$ being identical to or at least partially different from the area of the previous coupling step, with a saccharide moiety $SM_1$, $SM_2$, $SM_3$, $SM_4$..., $SM_m$ having at least one hydroxyl function protected by a protecting group P, said saccharide moiety being identical to or different from the saccharide moiety coupled during the previous coupling step, each coupling step a) being followed by a sub-step of removal of the protecting group P from at least one hydroxyl function of the saccharide moiety coupled during the previous coupling step;

d) deprotecting the hydroxyl functions of the saccharide type molecules that are still protected with a protecting group P.

The compounds of formula (I) which are used to passivate the hydroxyl functional groups of the surface of the solid support are compatible with the conditions used during the coupling steps of the different saccharide moieties. They are then not removed from the surface during the sugar synthesis because they are not sensible to the conditions used to remove the protective group P of the hydroxyl functions of the saccharides moieties. Therefore, the hydroxyl functional groups that have been passivated with compounds of formula (I) remain protected all along the sugar synthesis allowing the manipulation of a support having a very weakly reactive surface, thus improving signal/noise ratio.

Therefore, by this process, it is possible to obtain easily, in a reproducible manner and at an acceptable cost, glycochips exhibiting an improved signal/noise ratio allowing sensitive screening of saccharide or protein molecules and/or the detection of interactions with proteins of interest.

The nature of the solid supports that can be used according to the present invention is not critical. However, they are preferably chosen from supports based on glass, on silica or on any other material known to a person skilled in the art as being able to be modified by hydroxyl functional groups. These solid supports have at least one flat or nonflat and smooth or structured surface and can, for example, be provided in the form of a slide, flat plate, plate with wells, capillary or porous or nonporous bead.

According to the invention, it is possible to use either supports having a surface already bearing hydroxyl functional groups either as supports not naturally bearing hydroxyl functional groups and thus necessitating a preliminary step of hydroxylation of its surface with hydroxyl functional groups.

In this case, the method of the invention comprises a preliminary step of hydroxylation of the surface that can be performed by reacting at least selected regions of the surface of a solid support with a solution, in an organic solvent, of a spacer bearing at least one terminal hydroxyl functional group to obtain a surface modified by hydroxyl functional groups on said at least selected regions.

Advantageously, the spacer bearing at least one terminal hydroxyl functional used to modify the surface of the solid support is preferably chosen among silanizing agents bearing at least one hydroxyl functional group at one of their extremities. Such silanizing agents are more particularly chosen among compounds of following formulas (II-a) and (II-b) :

$$M-(CH_2)_x-O-R_6 \quad \text{(II-a)}$$

and

$$M-(CH_2)_x-R_7 \quad \text{(II-b)}$$

in which:

- M represents a silanized group $-Si(R_3)_3$, $-SiR_3(R_4)_2$ or $-SiR_3R_4$ and $R_5$, in which, $R_3$, $R_4$ et $R_5$, each independently, represent a hydrogen or a halogen atom such as fluorine and chlorine atoms, a $(C_1-C_4)$ alkoxy radical, a $(C_1-C_4)$ alkyl radical or a chloro$(C_1-C_4)$ alkyl radical;
- x is an integer ranging from 1 to 20 inclusive;
- $R_6$ represents a hydroxyl function protecting group;
- $R_7$ represents a precursor of a group containing at least one hydroxyl function such as an epoxide group.

The different hydroxyl protecting groups mentioned for $R_6$ are for example chosen among ether groups such as methoxymethylether (MOM) and di(p-methoxyphenyl) methylether (DMT) ; acetyl group, and more generally all protective group for a hydroxyl function such as those mentioned by T.W. Greene *et al.*, 2nd edition, Wiley Interscience.

Amongst such silanizing agents, one can mention in particular mention 5, 6-epoxyhexyltriethoxysilane and trifluoromethoxyundecanetrimethoxysilane.

The organic solvent used during the step of silanization can be chosen for example among trichloroethylene and toluene; trichloroethylene being particularly preferred.

The nature of the saccharide moiety that can be used in step a) and c) will depends of the final nature of the saccharide type molecules desired for the glycochip.
The saccharide moiety can be chosen among:

i) monosaccharides and in particular from glucosamine, azidoglucosamine, D-ribose, D-xylose, L-arabinose, D-glucose, D-galactose, D-mannose, 2-deoxyribose, L-fusose, N-acetyl-D-glucosamine, N-acetyl-D-galactosamine, N-acetylneuraminic acid, D-glucuronic acid, L-iduronic acid, D-sorbitol, D-mannitol, glucosides and the like;
ii) diholosides formed from the combination of two monosaccharides joined together by a glycosidic bond, such as sucrose, lactose, maltose, trehalose and cellobiose, glucopyranosides and the like;
iii) oligosaccharides having from 3 to 9 saccharidic units and in particular from such as fragments of heparan sulfates, saccharide fragments of heparin, of chondroitin and of dermatan sulfates, Lewis antigens and the like.

As previously mentioned, at least one hydroxyl function of the saccharide moieties is protected with a protecting group P. These protective groups are well known to a person skilled in the art and are fully described in the work by T. W. Greene et al., "Protective Groups in Organic Chemistry", Second Edition, A Wiley-Interscience Publication, 1991.
In addition, one or more of the amine functional groups and/or the carboxyl group of the saccharide moieties can also be protected by one or more protective groups. These protective groups are also well known to a person skilled in the art and are fully described in the work by T. W. Greene *et al*., previously cited.
According to an advantageous form of the present invention, these protective groups are chosen from the following groups: acetyl; p-methoxybenzyl; aryl and in particular the aryl groups substituted by an R radical chosen from alkyl chains having from 1 to 40 carbon atoms; 2, 2, 2-trichloroethyloxycarbonyl (Troc); benzyloxycarbonyl (Z); trichloroacetamidate (TCA); *tert*-butyloxycarbonyl (BOC) and fluoranylmethoxycarbonyl (Fmoc). They can also be chosen among the same protective groups that those mentioned as example for $R_6$ in compounds of formulas (II-a) and (II-b).
The solvent used during steps a) and c) can be chosen among dichloromethane, chloroform, acetonitrile, diethylether and toluene.
According to the invention, the halogen atom designated for $Y_1$, $Y_2$ and X in compounds of formula (I) can be chosen among chlorine, iodine, bromide and fluor atoms, the chlorine and fluorine atoms being preferred.
Among the $C_1$-$C_4$ alcoxy radicals mentioned for $Y_1$ and $Y_2$ in compounds of formula (I), one can mention the methyloxy, ethyloxy, propyloxy, n-butyloxy and *t*-butyloxy groups; the methyloxy group being preferred.
Among the $C_1$-$C_5$ alcoxy radicals mentioned for X in compounds of formula (I) one can mention the methyloxy, ethyloxy, propyloxy, n-butyloxy, *t*-butyloxy and n-pentyloxy groups; the methyloxy group being preferred.
Among linear and branched $C_1$-$C_4$ alkyl radicals designated for $Y_1$, $Y_2$ and X in compounds of formula (I), one can mention methyle, ethyle, propyle, n-butyle and *t*-butyle radicals.
Among thio($C_1$-$C_4$) alkyl radicals designated for $Y_1$, $Y_2$ and X in compounds of formula (I), one can mention methylthio, ethylthio, propylthio, n-butylthio and *t*-butylthio radicals; the methylthio and ethylthio radicals being preferred.
Among trifluoro($C_1$-$C_4$) alkyl radicals mentioned for $Y_1$ and $Y_2$ in compounds of formula (I), the trifluoromethyle radical is particularly preferred.
Among aryl, thioaryl and selenoaryl groups mentioned for $Y_1$, $Y_2$ and X in compounds of formula (I), the phenyl, thiophényle and selenophenyle groups are particularly preferred.
According to a preferred embodiment of the present invention, the compounds of formula (I) are selected in the group consisting of compounds in which

i) n = 1, one of $Y_1$ and $Y_2$ is a hydrogen atom and the other of $Y_1$ and $Y_2$ is a hydrogen or a halogen atom, a $C_1$-$C_4$ alcoxy radical, a thio($C_1$-$C_4$) alkyle radical or a cyano or an azido group and X is a halogen atom or a trichloroacetimide or a thio($C_1$-$C_4$) alkyl group;
ii) n = 1, $Y_1$ and $Y_2$ are identical and represent a $C_1$-$C_4$ alcoxy radical or a $C_1$-$C_4$ alkyle radical and X represents a halogen atom or a trichloroacetimide group or a thio($C_1$-$C_4$) alkyl group;
iii) n = 2, one of $Y_1$ and $Y_2$ is a hydrogen atom and the other of $Y_1$ and $Y_2$ is a hydrogen or a halogen atom, a $C_1$-$C_4$ alcoxy radical or a cyano or an azido group and X is a trichloroacetimide group;
iv) n = 2, $Y_1$ and $Y_2$ are identical and represent a $C_1$-$C_4$ alcoxy radical and X represents a trichloroacetimide group.

Among specific compounds of formula (I), the following compounds are particularly preferred:

- 2, 2, 2-trichloroacetimidic acid benzyl ester (X = trichloroacetimidate, $Y_1$ = $Y_2$ = H, n = 1);
- benzyl chloride (X = Cl, $Y_1$ = $Y_2$ = H, n = 1);
- benzyl bromide (X = Br, $Y_1$ = $Y_2$ = H, n = 1);
- 2-(trifluoromethyl) benzyl bromide (X = Br, $Y_1$ = H, $Y_2$ = $CF_3$, n = 1);
- 3, 5-di-(*tert*-butyl) benzyl bromide (X = Br, $Y_1$ = $Y_2$ = *tert*-butyl, n = 1); and

- 4-(methylthio) benzyl chloride (X = Cl, $Y_1$ = H, $Y_2$ = $CH_3S$, n = 1).

Compounds of formula (I) are commercially available or can be easily prepared according to the methods described in particular in "Protective groups in organic synthesis", Third edition, Theodora W. Green, Peter GM Wuts Copyright 1999, John Wiley & Sons, Inc.

According to a particular and preferred embodiment of the invention, the whole surface of the solid support is modified by surface hydroxyl functional groups. In this case, step a) is preferably preceded by a masking/unmasking step (M/U step) comprising the following sub-steps:

1) depositing at least one protection polymer on at least one selected region of the hydroxylated surface by microdeposition of drops of said polymer in solution in an organic solvent to form caps of solid polymer on the selected region(s) after evaporation of said solvent,
2) protecting the hydroxyl functions of the uncapped regions of the surface of the solid support with at least one compound of formula (I) as defined above, and
3) removal of the solid caps of protection polymer on the selected region(s) previously by dissolution said solid caps in an organic solvent.

This particular embodiment of the invention is well suited to solid supports in the form of plates having a plurality of microwells, those microwells corresponding to the selected regions that are capped with the protection polymer.

This particular embodiment of the invention is advantageous because it avoids the further mechanical addressing of selected regions during step a) and allows the subsequent coupling of saccharide moiety during step c) only in selected regions by the simple immersion of the whole surface of the solid support in a solution of said saccharide moiety.

According to another particular embodiment of the invention, selected regions of the surface of the solid support can also be masked by at least one protection polymer according to a masking/unmasking step which is performed before step a) and/or before and between each step c). Such a masking/unmasking step makes possible the synthesis of different saccharide sequences onto the same surface by successive immersions of the whole support in solutions of different saccharide moieties each separated by a masking/unmasking step on selected regions of the surface of the solid support.

According to this embodiment the M/U step is split into at least two sub-steps, a masking sub-step being performed before step a) and/or before and between each step c) and then an unmasking sub-step being performed after step a) and/or after each step c). This M/U step then comprises the following sub-steps:

1) a masking sub-step of depositing at least one protection polymer on at least one selected region of the surface of the solid support by microdeposition of drops of said polymer to form caps of solid polymer on the selected region (s) after evaporation of said solvent,
2) the coupling of a first saccharide moiety according to step a) as described before and/or the coupling of a further saccharide moiety according to step c) as described before, and
3) an unmasking sub-step of removal of the solid caps of protection polymer on the selected region(s) by dissolution said solid caps in an organic solvent.

The protection polymer can be selected from the group formed by polymers of polyvinyl alcohols, polystyrenes, polyvinyl carbazoles, polyimides and derivatives thereof, such polymers being neither soluble in the solvents used for reacting during the hydroxylation of the surface of the support (before step a)) nor in the solvent(s) used during the coupling of the saccharides moieties (steps a) and c)).

Among these polymers, polyhydroxystyrenes which are not soluble in dichloromethane are particularly preferred.

At the end of the masking sub-step, annealing of the support at a temperature of 50 to 80°C is generally performed to improve the adhesion of the protection polymer caps while accelerating the evaporation of the solvent.

The organic solvent used during the unmasking sub-step to dissolve the caps of protection polymer has to be inert with respect to the saccharides moieties already grafted on the surface support. This solvent is preferably chosen in the group comprising tetrahydrofuran, acetonitrile, ethanol, methanol, acetone and dimethylsulfoxide (DMSO).

According to another particular embodiment of the invention a passivation step of the unreacted hydroxyl functions present on saccharide moieties already grafted on the surface of the solid support and which have not reacted with a further saccharide moiety during a subsequent coupling step, is performed with at least one compound of formula (I) between each reiteration of step c) of coupling of a saccharide moiety. These repeated passivation steps are useful to stop the growth of truncated saccharide sequences during the manufacturing of the glycochip.

At the end of the synthesis, the process of the invention preferably comprises a further step of activation of the saccharide type molecules, this activation step being particularly useful for glycoaminoglycans and to allow the

recognition between the saccharide type molecules present on the support and proteins.

This activation step consists in removing the different protecting groups present on the hydroxyl/amine/carboxyle functional groups of the saccharide moieties. The removing of protective groups will of course be adapted to their nature as is it well known by the one skilled in the art. Therefore, the activation can be performed by deacetylation, debenzylation, etc, depending on the nature of the protective groups. As an example, when the protective group is a p-methoxybenzyl group, the activation step is preferably performed by immersion of the support in tetrahydrofuran during about 15 minutes at room temperature.

Another subject matter of the present invention is thus the solid support comprising at least one surface functionalized by one or more saccharide type molecules, **characterized in that** said support is obtained according to the manufacturing process of the invention.

Such supports constitute glycochips which are, for example, capable of being used for the identification, by screening, of saccharide molecules and in particular of oligosaccharide sequences which recognize a specific protein of advantage, for example using the method described in international application WO-A-03/008927.

Conversely, the solid support in accordance with the present invention can also be used for the identification, by screening, of ligands, for example of protein ligands which recognize a saccharide of advantage.

Therefore, an additional subject of the invention is the use of at least one solid support as defined above for the identification, by screening, of saccharide or protein molecules, or for the study of saccharides/proteins interactions.

Consequently, a final subject matter of the present invention is a process for screening saccharide molecules and in particular oligosaccharide sequences or respectively protein ligands, **characterized in that** it comprises at least one stage in which a solid support comprising at least one surface functionalized by at least one saccharide type molecule and prepared according to the invention manufacturing process as defined above is brought into contact with a solution including one or more potential saccharide molecules, in particular oligosaccharide molecules, or respectively one or more potential proteins.

In these specific applications, the functionalized solid supports in accordance with the present invention make it possible to optimize the screening processes by avoiding or limiting any unspecific absorption of molecules other than the molecules of interest whose immobilization is desired on the surface of the support and thus to have available more effectively and more rapidly molecules with a therapeutic or biotechnological aim.

In addition to the preceding provisions, the invention also comprises other provisions which will emerge from the description which will follow, which refers to an example of the preparation of a mannose chip, to an example of the preparation of glycoaminoglycans chip both in accordance with the process of the invention and from the attached figures on which:

- Figure 1 is a picture showing the fluorescence observed after a mannose recognition by lectine on a mannose chip **MC1** prepared according to the manufacturing process of the invention, i.e. including a passivation step with a compound of formula (I) as defined above, comparatively to the fluorescence observed in the same conditions on a mannose chip **MC2** prepared according to a manufacturing process not forming part of the present invention because including a passivation step with a benzyl compound not falling within the scope of formula (I) as defined above;
- Figure 2 is the reaction scheme of the preparation process of a glycoaminoglycans chip including protection polymer masking steps;
- Figure 3 is a picture showing the fluorescence observed after a glycoaminoglycan recognition by the HGF protein on a glycoaminoglycan chips (**GAG-C1** and **GAG-C'1**) prepared according to the manufacturing process of the invention, i.e. including a passivation step with a compound of formula (I) as defined above, comparatively to the fluorescence observed in the same conditions on glycoaminoglycan chips **GAG-C1** and **GAG-C'1**) prepared according to a manufacturing process not forming part of the present invention because including a passivation step with a benzyl compound not falling within the scope of formula (I) as defined above.

It should be clearly understood, however, that these examples are given solely by way of illustration of the subject matter of the invention, of which they do not under any circumstances constitute a limitation.

**<u>EXAMPLE 1: PREPARATION OF A GLYCOCHIP ACCORDING TO THE INVENTION MANUFACTURING PROCESS - COMPARISON WITH A GLYCOCHIP PREPARED BY A PROCESS NOT FORMING PART OF THE PRESENT INVENTION</u>**

In this example, a mannose chip **MC1** obtained by the preparation method according to the present invention has been prepared, using a solution of a mannose derivative as saccharide moiety and benzyl-2, 2, 2-trichloroacetimidate (Bn-OTCA) as passivation compound of formula (I). For the purpose of comparison, a mannose chip **MC2** (not forming part of the invention) has also been prepared using the same mannose derivative as saccharide moiety but replacing

Bn-OTCA with a passivation compound not falling within the scope of compounds of formula (I), namely 2, 3, 4, 6-tetra-O-benzyl-α-D-glucopyranosyl trichloroacetimidate (Bn-glucose-OTCA).

**1) Materials, Method and Reagents**

a) Sugar solution:

A solution of 10 mg of a mannose derivative **(Man-6)** having the following formula:

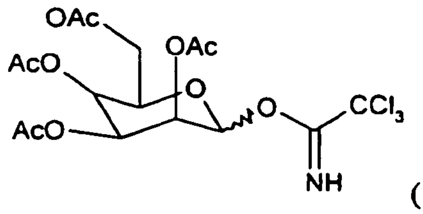

**(Man-6)**

in 1 ml of dichloromethane has been prepared.

This mannose derivative can be prepared starting from D-mannose according to the following reaction scheme A:

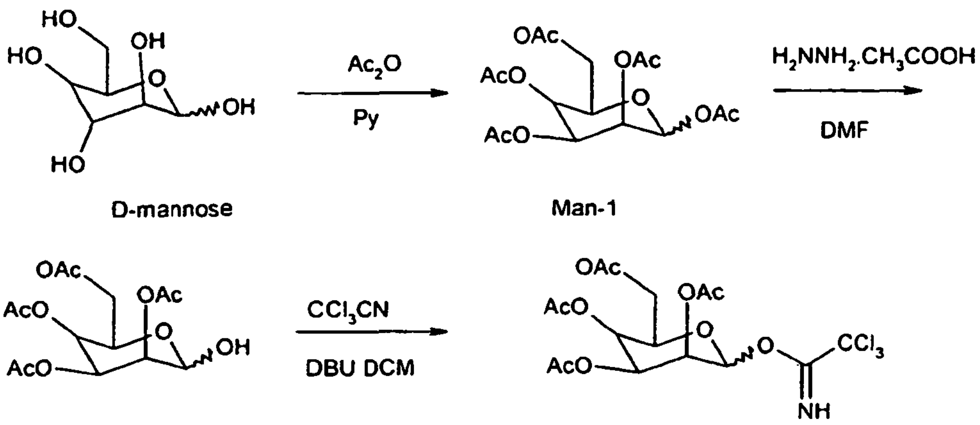


D-mannose

Man-1

Man-1-(OH-1)

Man-6

**SCHEMA A**

in which Ac represents acetyl, and wherein D-mannose is first acetylated with anhydride acetic ($Ac_2O$) in the presence of pyridine (Py) to give acetylated D-mannose (Man-1) (yield 90%) which is, in a second step deacetylated in the anomeric position in dimethylformamide (DMF) in the presence of hydrazine ($H_2NNH_2$) and acetic acid ($CH_3COOH$) to lead to 1-OH-acetylated D-Mannose (Man-1-(OH-1) (yield 77%) which is then converted in Man-6 in the presence of trichloroacetonitrile ($CCl_3CN$) in dichloromethane (DCM), using diazabicyclo-undecene (DBU) as catalyst for the reaction (yield 65%).

The sugar solution was then prepared by dissolving 10 mg of Man-6 in 1 ml of DCM.

b) Passivation solutions

The following passivation solutions were used:

- Passivation solution **PS1** consisting of 100 μl of Bn-OTCA in 2 ml of DCM.
- Passivation solution **PS2** consisting of 10 mg of BN-glucose-OTCA dissolved in 2 ml of DCM.

c) Masking Polymer Solution

A masking polymer solution comprising 5 % by weight of polyhydroxystyrene (PHS) dissolved in dimethylsulfoxide (DMSO) was also prepared.

d) Mannose chip preparation

**MC1** and **MC2** were prepared according to the following process:

i) Hydroxylation of the surface of the chips

Two substrates consisting of structured thermal silicium oxide ($Si/SiO_2$), having a thickness of 500 nm, a dimension of 1x1 cm, comprising 24 microwells (650 μm in diameter, 15 μm in depth and a step of 1500 between microwells) were firstly hydrated by immersion into a sodium hydroxide solution (12 g NaOH/50 ml of water/ 50 ml of ethanol), stirred for 2 hours, rinsed with water, then with ethanol and dried at 80°C for 15 minutes.

The substrates were then silanised by immersion in a silane solution (137 μl of 5, 6-epoxyhexyltriethoxysilane in 30 ml of toluene and 430 μL of thiethylamine) for 1 night at 80°C. The substrates were then rinsed in ethanol, DCM, and chloroform under sonication for 5 minutes before being baked at 110°C for 3 hours.

At the end of the silanisation step, the substrates were immersed into water with 5 % sulphuric acid solution in order to transform the epoxide moiety of the silane into the corresponding diol, stirred at room temperature for 2 hours, rinsed with water, ethanol, and then in water under sonication for 5 minutes.

ii) Masking_ step

The wells of each substrate were masked by injecting 30 drops by well of the Masking Polymer Solution. The substrates were baked at 80°C for 2 minutes to solidify the PHS. Each well was then again recovered with 20 drops, then 10 drops of the Masking Polymer Solution before being baked at 80°C for 2 minutes. Substrates comprising caps of solid PHS covering each well were thus obtained.

iii) Passivation step

One of the two masked substrates was immerged in 2 ml of the passivation solution **PS1,** the other was immerged in 2 ml of the passivation solution **PS2.** 40 μl of a solution made of 5 μl of trimethylsilyl trifluoromethanesulfonate (TMSOTf) in 100 μl of DCM were then added to each passivation solution. The substrates were left immersed in the passivation solutions for 1 hour under stirring at room temperature. The substrates were then rinsed with DCM, chloroform, ethanol and finally with chloroform under sonication for 5 min before being dried.

iv) Removing PHS caps

The caps of solidified PHS were removed by immersing the substrates in tetrahydrofuran. After a sonication step during 3 min., the substrates were rinsed with ethanol and dried.

v) Sugar coupling step

The substrates were immerged in the sugar solution and 10 μL of a solution made of 2 μL of TMS-OTf in 100 μL of DCM were added to each immerged substrate. The reaction mediums were stirred at room temperature for 30 min. Substrates were then rinsed with DCM, chloroform, ethanol and finally with chloroform under sonication for 5 min. The substrates were immerged in 40 μl of a 1 M sodium methanolate solution in methanol and stirred at room temperature for 30 min. Finally, the substrates were rinsed with methanol and with ethanol under sonication for 5 min.

vi) Mannose recognition by lectine

The substrates were immersed in a solution consisting of 30 mg of bovine serum albumine (BSA) in 5 ml of buffer A (Buffer A = Phosphate Buffered Saline (PBS) at 0.01 M, pH 7, 4 with 0.05% of Tween® 20) and stirred at room temperature for 1 hour.

After a rinsing step with buffer A, the substrates were immerged in a lectine solution (Concanavalin A from *Canavalia ensiformis* (Jack bean) biotin conjugate, Type IV, sold as a lyophilized powder under reference C2272 by the Company Sigma-Aldrich : 5 μL in 2 ml of Buffer B (Buffer B = PBS at 0.01 M with $Ca^{2+}$ 0.01 mM and $Mn^{2+}$ 0.1 mM)).

The substrates were left at 37°C for 1 hour.

After a new rinsing step with buffer A, substrates were immerged in a solution of stretavidine-Cy3 (2 μ of Cy3-streptavidine in 5 μL of Buffer R (Buffer R = PBS at 0.01 M with NaCl 0.5M and 0.05 % of Tween® 20)). The substrates were left at room temperature in the dark for 20 minutes, then rinsed with Buffer A and dried.

vii) Measure of the fluorescence signal

The scans of the substrate have been made with the GeneTAC ® LS IV Biochip Analyzer (Genomic Solutions®), which is a laser-based, high-throughput biochip imager and analyser.

FITC lecture ($\lambda_{excitation}$ = 498 nm ; $\lambda_{emission}$ = 518 nm)

Laser power PW = 42 %.

**2) Results**

Corresponding pictures of the results thus obtained are presented on the annexed Figure 1.

Theses results show that the process according to the present invention, i.e. wherein the passivation of the hydroxyl functions of the substrate is performed with a compound of formula (I) leads to a mannose chip **(MC1)** exhibiting an improved signal/noise ratio when compared to the image obtained when scanning the mannose chip **(MC2)** prepared using a passivation solution comprising a compound not falling within the scope of formula (I).

**EXAMPLE 2: PREPARATION OF A GLYCOAMINOGLYCAN CHIP ACCORDING TO THE INVENTION MANUFACTURING PROCESS - COMPARISON WITH A GLYCOAMINOGLYCAN PREPARED BY A PROCESS NOT FORMING PART OF THE PRESENT INVENTION**

In this example, a glycoaminoglycans chip **(GAG-C1)** obtained by the preparation process according to the present invention has been prepared, using the passivation solution **PS1** as prepared in example 1 above and a sugar unit of specific structure defined below.

For the purpose of comparison a glycoaminoglycans chip not forming part of the invention **(GAG-C2)** has also been prepared using the passivation solution **PS2** as prepared in example 1 above.

**1) Materials and method**

a) Sugar solution:

Methyl 2-*O*-acetyl-3-*O*-benzyl-4-*O*-(4-methoxybenzyl) -α-L-idopyranosyluronate) -(1→4) -*O*-6-*O*-acetyl-2-azido-3-*O*-benzyl-2-deoxy-D-glucopyranoside trichloroacetimidate has been used as sugar unit for the solid supported synthesis of corresponding glycoaminoglycans. This sugar unit **(GAG)** has the following chemical structure:

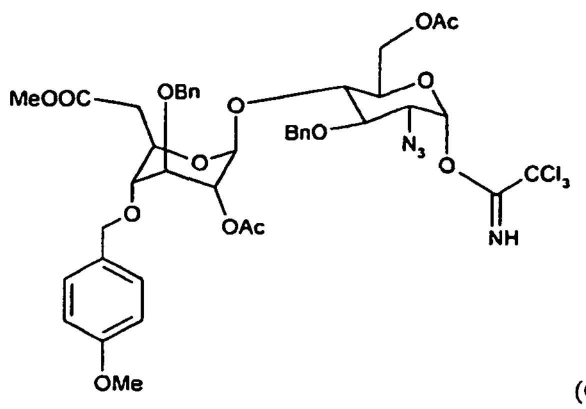

**(GAG)**

The synthesis of this sugar unit has already been reported in the articles by Bonnaffé et al., European Journal of Organic Chemistry, 2003, 3603-3620 and A. Lubineau et al., Chemical European Journal, 2004, 10, 4265-4282.
The sugar solution was prepared by dissolving 8 mg of **(GAG)** in 500 ml of DCM.

b) Passivation solutions

Passivation solutions **PS1** and **PS2** as prepared here above in example 1 were used.

c) Masking Polymer Solution

We used the masking polymer solution comprising 5 % by weight of polyhydroxystyrene (PHS) dissolved in dimethylsulfoxide (DMSO) as in example 1 above.

d) Glycoaminoglycan chip preparation

**GAG-C1** and **GAG-C2** were prepared according to the following process: The reaction scheme (Scheme B) to prepare the glycoaminoglycan chip is illustrated on the attached figure 2.
In this example, we have used the same structured silicium oxide substrates as in example 1.

i) Surface hydroxylation of the chips

The whole surface of the chips was firstly hydroxylated according to the process disclosed in example 1 above.

ii) Masking step

The wells of each chip were masked with a PHS solution as described in example 1 above.
Chips comprising caps of solid PHS covering each well were thus obtained.

iii) Removing PHS caps

The caps of solidified PHS were removed from each well according to the process described in example 1 above.

iv) Passivation step

The passivation steps using the passivation solution **PS1** on one chip, and the passivation solution **PS2** on the other chip was performed as described in example 1 above.

v) Glycoaminoglycan synthesis

The growth of GAG oligomers on the chips was performed according to the following step cycle:

- PHS masking step: the wells of a determined selected region of the chip were capped according to the process described in example 1 above.
- Passivation step:
  The passivation steps using the passivation solution **PS1** on one chip, and the passivation solution **PS2** on the other chip was performed as described in example 1 above.
- GAG coupling step:
  8 mg of GAGs monomer in 500 μL DCM were injected in an Ichigo-ki ® automat synthesizer. The synthesis chamber was maintained at -10°C. The TMSOTf solution was then injected (1 μL in 500 μL and left at -10°C for 1 hour. The substrates were then rinsed in DCM.

The GAG coupling step was then repeated twice to lead to hexamers.

vi) Activation of the glycoaminoglycans

• Methyl ester exchange / Deacetylation

The chips were then immersed in a solution of 40 μL of sodium benzylate (BnONa) in 2 ml of benzyl alcohol

(BnOH). The reaction medium was stirred at room temperature for 1 night. The chips were then rinsed with methanol and ethanol under sonication for 5 min.

• Azide reduction

The chips were then immersed in a solution of 1.2 ml propanedithiol / 1.5 ml triethylamine ($Et_3N$) / 6 ml methanol. The reaction medium was stirred at room temperature for 2 days. The chips were then rinsed with methanol and ethanol under sonication for 5 min.

• Sulfation

The chips were immersed in a solution of 200 mg of sulfur trioxide in 10 ml of pyridine. The reaction medium was stirred at room temperature for 1 day and then left at a temperature of 55°C for 1 day. The chips were then rinsed with ethanol, water and finally with ethanol under sonication for 5 min.

• Debenzylation

The chips were put in a flask and immersed in a solution of 10 mg of Pd-Polyvinylpyrrolidone (Pd-PVP) nanoparticules in 15 ml of propanol and 15 ml of water. The flask was closed and hydrogen gas at atmospheric pressure was then added during 15 min. The flask was kept closed and maintained at a temperature of 45°C for 1 night. Hydrogen gas at atmospheric pressure was again added during 15 min and the flask was again kept closed and maintained at a temperature of 45°C for 1 night. The chips were then rinsed with ethanol, water and finally with ethanol under sonication for 5 min.

vii) GAGs recognition by the HGF protein

The chips were immerged in a BSA solution comprising 30 mg of BSA in 5 ml of Buffer A as described above in example 1. The reaction medium was stirred at room temperature for 1 hour. After the chips were rinsed with Buffer A and then several drops of a HGF solution (Hepatocyte Growth Factor, Human H9661- 5 μg) at a concentration of 70 nM in PBS were applied on the chips. The chips were covered with a plastic patch and left at room temperature for 2 hours. After a washing with PBS, the chips were immersed in an anti-HGF (Monoclonal anti-HGF Antibody, Sigma, H1896, 5 mg in 1 ml of Buffer B) solution diluted 50 times in a 0.1 % BSA buffer in PBS, and left in that solution for 1 hour at a temperature of 38°C. At the end of that incubation, the chips were washed with Buffer A and then immersed in a Fluoresceine Iso Thio Cyanate (FITC) labeled anti anti-HGF solution (Antimouse IgG FITC, Sigma, F5687-5 mL (solution further diluted 100 times with 0.1% BSA buffer in PBS). The chips were again incubated during 1 hour at 38°C. At the end of the incubation period, the chips were washed with Buffer A, wet with PBS and scanned with the GeneTAC ® LS IV Biochip Analyzer as used in example 1 above.

The same experiment has been performed in identical conditions but with a HGF solution at 200 nM in PBS and lead to **GAG-C'1** and **GAG-C'2.**

FITC lecture ($\lambda_{excitation}$ = 498 nm ; $\lambda_{emission}$ = 518 nm)

Laser power PW = 42 %.

**2 Results**

The results are reported on the attached figure 3.

The results show that the glycoaminoglycans chips (**GAG-C1** and **GAG-C'1**) obtained by the process according to the present invention, i.e. using Bn-OTCA as passivation compound of formula (I) exhibit an improved signal/noise ratio when compared to the image obtained when scanning the glycoaminoglycans chips (**GAG-C2 and GAG-C'2**) prepared using a passivation solution comprising a compound not falling within the scope of formula (I).

## Claims

1. Method for the *in situ* synthesis of saccharide type molecules onto the surface of a solid support), said surface being modified with hydroxyl functional groups, wherein said method comprises the following steps:

a) coupling, on said surface hydroxyl functional groups, a first saccharide moiety $SM_1$ having at least one hydroxyl function protected with a protecting group P, by contacting at least one area $A_1$ of said surface with a solution, in a solvent, of said first saccharide moiety $SM_1$;
b) passivating the unreacted surface hydroxyl functional groups by contacting the surface of the solid support with at least one compound of formula (I) below:

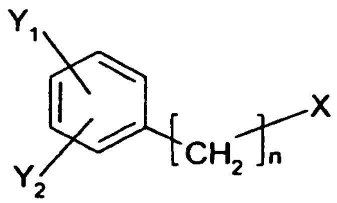

(I)

in which:

- $Y_1$ and $Y_2$, which may be identical or different, represent a hydrogen atom, a halogen atom, a $C_1$-$C_4$ alcoxy radical, a linear or branched $C_1$-$C_4$ alkyl radical, a thio($C_1$-$C_4$) alkyl radical, a nitro group, an azido group, a trifluoro($C_1$-$C_4$) alkyl radical, a cyano group or an aryl ring;
- n is an integer ranging from 1 to 4 inclusive;
- X is selected from the group consisting of a halogen atom, a trichloroacetimide group, a xanthate group -SC=SOR$_1$ in which $R_1$ represents a linear or branched $C_1$-$C_4$ alkyl radical, a thio($C_1$-$C_4$) alkyl group, a thioaryl group, a phosphate group, a phosphite group, a seleno($C_1$-$C_4$) alkyl group, selenoaryl group, a $C_1$-$C_5$ alcoxy radical or a sulfoxide group -S(O) -R$_2$ in which $R_2$ represents linear or branched $C_1$-$C_4$ alkyl radical or an aryl ring;

with the proviso that when one of $Y_1$ and $Y_2$ represents hydrogen and the other of $Y_1$ and $Y_2$ is a methoxy radical, then the methoxy radical is not in the para position with regards to the carbon atom bearing the $-(CH_2)_n$-X chain;
c) reiterating coupling steps a) until the obtaining of a plurality of saccharide type molecules of determined saccharide sequences wherein each coupling steps a) is performed on at least one selected area $A_2$, $A_3$, $A_4$..., $A_m$ of the surface, said area $A_2$, $A_3$, $A_4$..., $A_m$ being identical to or at least partially different from the area of the previous coupling step, with a saccharide moiety $SM_1$, $SM_2$, $SM_3$, $SM_4$..., $SM_m$ having at least one hydroxyl function protected by a protecting group P, said saccharide moiety being identical to or different from the saccharide moiety coupled during the previous coupling step, each coupling step a) being followed by a sub-step of removal of the protecting group P from at least one hydroxyl function of the saccharide moiety coupled during the previous coupling step;
d) deprotecting the hydroxyl functions of the saccharide type molecules that are still protected with a protecting group P.

2. The method according to claim 1, wherein it uses a support which does not naturally bear hydroxyl functional groups and wherein it comprises a preliminary step of hydroxylation of the surface that is performed by reacting at least selected regions of the surface said support with a solution, in an organic solvent, of a spacer bearing at least one terminal hydroxyl functional group to obtain a surface modified by hydroxyl functional groups on said at least selected regions.

3. The method according to claim 2, wherein the spacer bearing at least one terminal hydroxyl functional is a silanizing agent chosen among compounds of following formulas (II-a) and (II-b) :

$$M\text{-}(CH_2)_x\text{-}O\text{-}R_6 \qquad \text{(II-a)}$$

and

$$M\text{-}(CH_2)_x\text{-}R_7 \qquad \text{(II-b)}$$

in which:

- M represents a silanized group $-Si(R_3)_3$, $-SiR_3(R_4)_2$ or $-SiR_3R_4R_5$, in which, $R_3$, $R_4$ and $R_5$, each independently, represent a hydrogen or a halogen atom such as fluorine and chlorine atoms, a $(C_1\text{-}C_4)$ alkoxy radical, a $(C_1\text{-}C_4)$ alkyl radical or a chloro$(C_1\text{-}C_4)$ alkyl radical;
- x is an integer ranging from 1 to 20 inclusive;
- $R_6$ represents a hydroxyl function protecting group;
- $R_7$ represents a precursor of a group containing at least one hydroxyl function such as an epoxide group.

**4.** The method according to claim 3, wherein the silanizing agent is 5, 6-epoxyhexyltriethoxysilane or trifluoromethoxyundecanetrimethoxysilane.

**5.** The method according to any one of the preceding claims, wherein the saccharide moiety is chosen among monosaccharides, diholosides and oligosaccharides.

**6.** The method according to any one of the preceding claims, wherein the solvent used during steps a) and c) are chosen among dichloromethane, chloroform, acetonitrile, diethylether and toluene.

**7.** The method according to any one of the preceding claims, wherein compounds of formula (I) are selected in the group consisting of compounds in which

i) n = 1, one of $Y_1$ and $Y_2$ is a hydrogen atom and the other of $Y_1$ and $Y_2$ is a hydrogen or a halogen atom, a $C_1\text{-}C_4$ alcoxy radical, a thio$(C_1\text{-}C_4)$ alkyle radical or a cyano or an azido group and X is a halogen atom or a trichloroacetimide or a thio$(C_1\text{-}C_4)$ alkyl group;
ii) n = 1, $Y_1$ and $Y_2$ are identical and represent a $C_1\text{-}C_4$ alcoxy radical or a $C_1\text{-}C_4$ alkyle radical and X represents a halogen atom or a trichloroacetimide group or a thio$(C_1\text{-}C_4)$ alkyl group;
iii) n = 2, one of $Y_1$ and $Y_2$ is a hydrogen atom and the other of $Y_1$ and $Y_2$ is a hydrogen or a halogen atom, a $C_1\text{-}C_4$ alcoxy radical or a cyano or an azido group and X is a trichloroacetimide group;
iv) n = 2, $Y_1$ and $Y_2$ are identical and represent a $C_1\text{-}C_4$ alcoxy radical and X represents a trichloroacetimide group.

**8.** The method according to any one of the preceding claims, wherein compounds of formula (I) are chosen among 2, 2, 2-trichloroacetimidic acid benzyl ester; benzyl chloride; benzyl bromide; 2-(trifluoromethyl) benzyl bromide; 3, 5-di-(*tert*-butyl) benzyl bromide and 4-(methylthio) benzyl chloride.

**9.** The method according to any one of the preceding claims, wherein the whole surface of the solid support is modified by surface hydroxyl functional groups and wherein step a) is preceded by a masking/unmasking step comprising the following sub-steps:

1) depositing at least one protection polymer on at least one selected region of the hydroxylated surface by microdeposition of drops of said polymer in solution in an organic solvent to form caps of solid polymer on the selected region(s) after evaporation of said solvent,
2) protecting the hydroxyl functions of the uncapped regions of the surface of the solid support with at least one compound of formula (I) as defined above, and
3) removal of the solid caps of protection polymer on the selected region(s) previously by dissolution said solid caps in an organic solvent.

**10.** The method according to any one of the preceding claims, wherein selected regions of the surface of the solid support are masked by at least one protection polymer according to masking/unmasking step which is performed before step a) and/or before and between each step c).

**11.** The method of claim 10, wherein the masking/unmasking step is split into at least two sub-steps, a masking substep being performed before step a) and/or before and between each step c) and then an unmasking sub-step being performed after step a) and/or after each step c).

**12.** The method according to claim 11, wherein the masking/unmasking step comprises the following sub-steps:

1) a masking sub-step of depositing at least one protection polymer on at least one selected region of the surface of the solid support by microdeposition of drops of said polymer to form caps of solid polymer on the selected region(s) after evaporation of said solvent,
2) the coupling of a first saccharide moiety according to step a) as described in claim 1 and/or the coupling of a further saccharide moiety according to step c) as described in claim 1, and
3) an unmasking sub-step of removal of the solid caps of protection polymer on the selected region(s) by dissolution said solid caps in an organic solvent.

**13.** The method according to any one of claims 9 to 11, wherein the protection polymer is selected from the group formed by polymers of polyvinyl alcohols, polystyrenes, polyvinyl carbazoles, polyimides and derivatives thereof, such polymers being neither soluble in the solvents used for reacting during the hydroxylation of the surface of the support (before step a)) nor in the solvent(s) used during the coupling of the saccharides moieties (steps a) and c)).

**14.** The method according to claim 13, wherein the protection polymer is chosen among polyhydroxystyrenes which are not soluble in dichloromethane.

**15.** The method according to any one of claims 9 to 14, wherein the organic solvent used during the unmasking sub-step to dissolve the caps of protection polymer is chosen in the group comprising tetrahydrofuran, acetonitrile, ethanol, methanol, acetone and dimethylsulfoxide.

**16.** The method according to any one of the preceding claims, wherein a passivation step of the unreacted hydroxyl functions present on saccharide moieties already grafted on the surface of the solid support and which have not reacted with a further saccharide moiety during a subsequent coupling step, is performed with at least one compound of formula (I) as defined in claim 1 between each reiteration of step c) of coupling of a saccharide moiety.

**17.** The method according to any one of the preceding claims, wherein it comprises, at the end of the synthesis, a further step of activation of the saccharide type molecules.

**18.** The method according to claim 17, wherein the activation step consists in removing the different protecting groups present on the hydroxyl/amine/carboxyle functional groups of the saccharide moieties.

**19.** A solid support comprising at least one surface functionalized by one or more saccharide type molecules, **characterized in that** said support obtained according to the manufacturing process as defined in any one of the preceding claims.

**20.** Use of at least one solid support as defined in claim 19 for the identification, by screening, of saccharide or protein molecules or for the study of saccharides/proteins interactions.

**21.** A process for screening saccharide molecules or respectively protein ligands, **characterized in that** it comprises at least one stage in which a solid support comprising at least one surface functionalized by at least one saccharide type molecule and prepared according to the method as defined in any one of claims 1 to 18 is brought into contact with a solution including one or more potential saccharide molecules or respectively one or more potential protein.

## Patentansprüche

**1.** Verfahren für die in-situ-Synthese von Molekülen des Saccharid-Typs an der Oberfläche eines festen Trägers, wobei die Oberfläche mit funktionellen Hydroxylgruppen modifiziert ist, wobei das Verfahren die folgenden Schritte umfasst:

a) Kuppeln einer ersten Saccharid-Gruppierung $SM_1$, die wenigstens eine Hydroxylfunktion hat, die mit einer Schutzgruppe P geschützt ist, an die funktionellen Oberflächen-Hydroxylgruppen, indem wenigstens ein Bereich $A_1$ der Oberfläche mit einer Lösung der ersten Saccharid-Gruppierung $SM_1$ in einem Lösungsmittel in Kontakt gebracht wird;
b) Passivieren der nicht-umgesetzten funktionellen Oberflächen-Hydroxylgruppen, indem die Oberfläche des festen Trägers mit wenigstens einer Verbindung der unten stehenden Formel (I) in Kontakt gebracht wird:

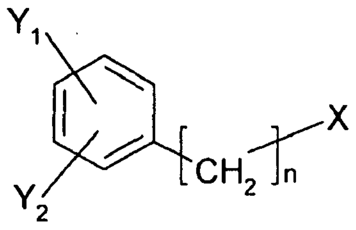

(I)

in der:

- $Y_1$ und $Y_2$, die identisch oder unterschiedlich sein können, ein Wasserstoffatom, ein Halogenatom, einen $C_1$-$C_4$-Alkoxy-Rest, einen linearen oder verzweigten $C_1$-$C_4$-Alkyl-Rest, einen Thio($C_1$-$C_4$) alkyl-Rest, eine Nitro-Gruppe, eine Azido-Gruppe, einen Trifluor($C_1$-$C_4$) alkyl-Rest, eine CyanoGruppe oder einen Aryl-Ring darstellen;
- n eine ganze Zahl im Bereich von 1 bis 4, einschließlich, ist;
- X ausgewählt ist aus der Gruppe, bestehend aus einem Halogenatom, einer Trichloracetimid-Gruppe, einer Xanthat-Gruppe -SC=SOR$_1$, worin $R_1$ einen linearen oder verzweigten $C_1$-$C_4$-Alkyl-Rest darstellt, einer Thio($C_1$-$C_4$) alkyl-Gruppe, einer Thioaryl-Gruppe, einer Phosphat-Gruppe, einer Phosphit-Gruppe, einer Seleno($C_1$-$C_4$) alkyl-Gruppe, Selenoaryl-Gruppe, einer $C_1$-$C_5$-Alkoxy-Rest oder einer Sulfoxid-Gruppe -S(O) -$R_2$, worin $R_2$ einen linearen oder verzweigten $C_1$-$C_4$-Alkyl-Rest oder einen Aryl-Ring darstellt;

mit der Maßgabe, dass, wenn eines von $Y_1$ und $Y_2$ Wasserstoff darstellt und das andere von $Y_1$ und $Y_2$ einen Methoxy-Rest darstellt, dann der Methoxy-Rest nicht in para-Position bezüglich des Kohlenstoffatoms, das die -$(CH_2)_n$-X-Kette trägt, ist;

c) Wiederholen von Kupplungsschritten a) bis zum Erhalten einer Vielzahl von Molekülen des Saccharid-Typs mit bestimmten Saccharid-Sequenzen, wobei jeder Kupplungsschritt a) an wenigstens einem ausgewählten Bereich $A_2$, $A_3$, $A_4$...$A_m$ der Oberfläche durchgeführt wird, wobei der Bereich $A_2$, $A_3$, $A_4$...$A_m$ identisch mit oder wenigstens teilweise verschieden von dem Bereich des vorherigen Kupplungsschrittes ist, wobei eine Saccharid-Gruppierung $SM_1$, $SM_2$, $SM_3$, $SM_4$...$SM_m$ wenigstens eine Hydroxylfunktion hat, die durch eine Schutzgruppe P geschützt ist, wobei die Saccharid-Gruppierung identisch mit oder verschieden von der Saccharid-Gruppierung ist, die während des vorherigen Kupplungsschrittes gekuppelt wurde, wobei auf jeden Kupplungsschritt a) ein Unterschritt der Entfernung der Schutzgruppe P von wenigstens einer Hydroxylfunktion der Saccharid-Gruppierung, die während des vorherigen Kupplungsschrittes gekuppelt wurde, folgt;

d) Entschützen der Hydroxylfunktionen der Moleküle des Saccharid-Typs, die noch mit einer Schutzgruppe P geschützt sind.

2. Verfahren gemäß Anspruch 1, wobei es einen Träger verwendet, der natürlicherweise keine funktionellen Hydroxylgruppen trägt, und wobei es einen vorausgehenden Schritt der Hydroxylierung der Oberfläche umfasst, der durchgeführt wird, indem wenigstens ausgewählte Regionen der Oberfläche des Trägers mit einer Lösung eines Spacers, der wenigstens eine terminale funktionelle Hydroxylgruppe trägt, in einem organischen Lösungsmittel umgesetzt werden, um eine Oberfläche zu erhalten, die mit funktionellen Hydroxylgruppen an wenigstens den ausgewählten Regionen modifiziert ist.

3. Verfahren gemäß Anspruch 2, wobei der Spacer, der wenigstens ein terminales funktionelles Hydroxyl trägt, ein Silanisierungsmittel ist, das aus Verbindungen der folgenden Formeln (II-a) und (II-b) ausgewählt ist:

$$M\text{-}(CH_2)_x\text{-}O\text{-}R_6 \qquad \text{(II-a)}$$

und

$$M\text{-}(CH_2)_x\text{-}R_7 \qquad \text{(II-b)}$$

worin:

- M eine silanisierte Gruppe -Si$(R_3)_3$, -Si$R_3(R_4)_2$ oder -Si$R_3R_4R_5$ darstellt, worin $R_3$, $R_4$ und $R_5$ jeweils unabhängig ein Wasserstoffatom oder ein Halogenatom, zum Beispiel Fluor- und Chloratome, einen ($C_1$-$C_4$) Alk-

oxy-Rest, einen ($C_1$-$C_4$) Alkyl-Rest oder einen Chlor($C_1$-$C_4$) alkyl-Rest darstellen;
- x eine ganze Zahl im Bereich von 1 bis 20, einschließlich, ist;
- $R_6$ eine Schutzgruppe der Hydroxylfunktion darstellt;
- $R_7$ einen Vorläufer einer Gruppe, die wenigstens eine Hydoxylfunktion enthält, zum Beispiel eine EpoxidGruppe, darstellt.

**4.** Verfahren gemäß Anspruch 3, wobei das Silanisierungsmittel 5, 6-Epoxyhexyltriethoxysilan oder Trifluormethoxyundecantrimethoxysilan wird.

**5.** Verfahren gemäß einem der vorangehenden Ansprüche, wobei die Saccharid-Gruppierung aus Monosacchariden, Diholosiden und Oligosacchariden ausgewählt wird.

**6.** Verfahren gemäß einem der vorangehenden Ansprüche, wobei das Lösungsmittel, das während der Schritte a) und c) verwendet wird, aus Dichlormethan, Chloroform, Acetonitril, Diethylether und Toluol ausgewählt wird.

**7.** Verfahren gemäß einem der vorangehenden Ansprüche, wobei Verbindungen der Formel (I) ausgewählt werden aus der Gruppe, bestehend aus Verbindungen, in denen

i) n = 1, eines von $Y_1$ und $Y_2$ ein Wasserstoffatom ist und das andere von $Y_1$ und $Y_2$ ein Wasserstoffatom oder ein Halogenatom, ein $C_1$-$C_4$-Alkoxy-Rest, ein Thio($C_1$-$C_4$) alkyl-Rest oder eine Cyano- oder eine Azido-Gruppe ist und X ein Halogenatom oder ein Trichloracetimid-Gruppe oder eine Thio($C_1$-$C_4$) alkyl-Gruppe ist;
ii) n = 1, $Y_1$ und $Y_2$ identisch sind und einen $C_1$-$C_4$-Alkoxy-Rest oder einen $C_1$-$C_4$-Alkyl-Rest darstellen und X ein Halogenatom oder eine Trichloracetimid-Gruppe oder eine Thio($C_1$-$C_4$) alkyl-Gruppe darstellt;
iii) n = 2, eines von $Y_1$ und $Y_2$ ein Wasserstoffatom ist und das andere von $Y_1$ und $Y_2$ ein Wasserstoffatom oder ein Halogenatom, ein $C_1$-$C_4$-Alkoxy-Rest oder eine Cyano- oder eine Azido-Gruppe ist und X eine Trichloracetimid-Gruppe ist;
iv) n = 2, $Y_1$ und $Y_2$ identisch sind und einen $C_1$-$C_4$-Alkoxy-Rest darstellen und X eine Trichloracetimid-Gruppe darstellt.

**8.** Verfahren gemäß einem der vorangehenden Ansprüche, wobei Verbindungen der Formel (I) ausgewählt werden aus 2, 2, 2-Trichloracetimidsäurebenzylester; Benzylchlorid; Benzylbromid; 2-(Trifluormethyl) benzylbromid; 3, 5-Di-(tert-butyl) benzylbromid und 4-(Methylthio) benzylchlorid.

**9.** Verfahren gemäß einem der vorangehenden Ansprüche, wobei die gesamte Oberfläche des festen Trägers durch funktionelle Oberflächen-Hydroxylgruppen modifiziert ist und wobei Schritt a) ein Maskierungs/Entmaskierungsschritt vorausgeht, der die folgenden Unterschritte umfasst:

1) Abscheiden wenigstens eines Schutzpolymers an wenigstens einer ausgewählten Region der hydroxylierten Oberfläche durch Mikroabscheidung von Tropfen des Polymers in Lösung in einem organischen Lösungsmittel unter Bildung von Kappen aus festem Polymer an der ausgewählten Region (an den ausgewählten Regionen) nach Verdampfung des Lösungsmittels,
2) Schützen der Hydroxylfunktionen der nicht verkappten Regionen der Oberfläche des festen Trägers mit wenigstens einer Verbindung der Formel (I), wie sie oben definiert ist, und
3) vorausgehende Entfernung der festen Kappen aus Schutzpolymer an der ausgewählten Region (den ausgewählten Regionen) durch Auflösen der festen Kappen in einem organischen Lösungsmittel.

**10.** Verfahren gemäß einem der vorangehenden Ansprüche, wobei ausgewählte Regionen der Oberfläche des festen Trägers durch wenigstens ein Schutzpolymer gemäß dem Maskierungs/Entmaskierungsschritt, der vor Schritt a) und/oder vor und zwischen jedem Schritt c) durchgeführt wird, maskiert werden.

**11.** Verfahren gemäß Anspruch 10, wobei der Maskierungs/Entmaskierungsschritt in wenigstens zwei Unterschritte aufgeteilt wird, einen Maskierungsunterschritt, der vor Schritt a) und/oder vor und zwischen jedem Schritt c) durchgeführt wird, und dann einen Entmaskierungsunterschritt, der nach Schritt a) und/oder nach jedem Schritt c) durchgeführt wird.

**12.** Verfahren gemäß Anspruch 11, wobei der Maskierungs/Entmaskierungsschritt die folgenden Unterschritte umfasst:

1) einen Maskierungsunterschritt des Abscheidens wenigstens eines Schutzpolymers an wenigstens einer

ausgewählten Region der Oberfläche des festen Trägers durch Mikroabscheidung von Tropfen des Polymers unter Bildung von Kappen des festen Polymers an der ausgewählten Region (den ausgewählten Regionen) nach Verdampfung des Lösungsmittels,
2) die Kupplung einer ersten Saccharid-Gruppierung gemäß Schritt a), wie in Anspruch 1 beschrieben, und/ oder die Kupplung einer weiteren Saccharid-Gruppierung gemäß Schritt c), wie in Anspruch 1 beschrieben, und
3) einen Entmaskierungsunterschritt der Entfernung der festen Kappen aus Schutzpolymer an der ausgewählten Region (an den ausgewählten Regionen) durch Auflösung der festen Kappen in einem organischen Lösungsmittel.

**13.** Verfahren gemäß einem der Ansprüche 9 bis 11, wobei das Schutzpolymer ausgewählt wird aus der Gruppe, die durch Polymere von Polyvinylalkoholen, Polystyrolen, Polyvinylcarbazolen, Polyimiden und Derivaten davon gebildet wird, wobei solche Polymere weder in den Lösungsmitteln, die zum Umsetzen während der Hydroxylierung der Oberfläche des Trägers (vor Schritt a)) verwendet werden, noch in dem Lösungsmittel (den Lösungsmitteln), das/die während der Kupplung der Saccharid-Gruppierungen (Schritte a) und c)) verwendet wird/werden, löslich sind.

**14.** Verfahren gemäß Anspruch 13, wobei das Schutzpolymer aus Polyhydroxystyrolen, die in Dichlormethan nicht löslich sind, ausgewählt wird.

**15.** Verfahren gemäß einem der Ansprüche 9 bis 14, wobei das organische Lösungsmittel, das während des Entmaskierungsunterschrittes verwendet wird, um die Kappen des Schutzpolymers zu lösen, aus der Gruppe, umfassend Tetrahydrofuran, Acetonitril, Ethanol, Methanol, Aceton und Dimethylsulfoxid, ausgewählt wird.

**16.** Verfahren gemäß einem der vorangehenden Ansprüche, wobei ein Passivierungsschritt der nicht-umgesetzten Hydroxylfunktionen, die an Saccharid-Gruppierungen vorliegen, die bereits an die Oberfläche des festen Trägers gepfropft sind und die nicht mit einer weiteren Saccharid-Gruppierung während eines nachfolgenden Kupplungsschrittes reagiert haben, mit wenigstens einer Verbindung der Formel (I), wie sie in Anspruch 1 definiert ist, zwischen jeder Wiederholung von Schritt c) der Kupplung einer Saccharid-Gruppierung durchgeführt wird.

**17.** Verfahren gemäß einem der vorangehenden Ansprüche, wobei es am Ende der Synthese einen weiteren Schritt der Aktivierung der Moleküle des Saccharid-Typs umfasst.

**18.** Verfahren gemäß Anspruch 17, wobei der Aktivierungsschritt in der Entfernung verschiedener Schutzgruppen, die an den funktionellen Hydroxyl/Amin/Carboxyl-Gruppen der Saccharid-Gruppierungen vorliegen, besteht.

**19.** Fester Träger, der wenigstens eine Oberfläche umfasst, die durch ein Molekül oder mehrere Moleküle des Saccharid-Typs funktionalisiert ist, **dadurch gekennzeichnet, dass** der Träger nach dem Herstellungsverfahren erhalten wird, wie es in einem der vorangehenden Ansprüche definiert ist.

**20.** Verwendung wenigstens eines festen Trägers, wie er in Anspruch 19 definiert ist, zur Identifikation, durch Screening, von Saccharid- oder Proteinmolekülen oder für die Untersuchung von Saccharid/Protein-Wechselwirkungen.

**21.** Verfahren zum Screening von Saccharid-Molekülen bzw. Proteinliganden, **dadurch gekennzeichnet, dass** es wenigstens eine Stufe umfasst, in der ein fester Träger, der wenigstens eine Oberfläche, die durch wenigstens ein Molekül des Saccharid-Typs funktionalisiert ist und nach dem Verfahren, wie es in einem der Ansprüche 1 bis 18 definiert ist, hergestellt wurde, in Kontakt mit einer Lösung gebracht wird, die ein potentielles Saccharid-Molekül oder mehrere potentielle Saccharid-Moleküle bzw. ein potentielles Protein oder mehrere potentielle Proteine umfasst.

## Revendications

**1.** Procédé de synthèse *in situ* de molécules de type saccharide sur la surface d'un support solide, ladite surface étant modifiée avec des groupes fonctionnels hydroxyle, ledit procédé comprenant les étapes suivantes :

a) le couplage, sur lesdits groupes fonctionnels hydroxyle de surface, d'un premier fragment saccharidique $SM_1$ possédant au moins une fonction hydroxyle protégée par un groupe protecteur P, par le contact d'au moins une zone $A_1$ de ladite surface avec une solution, dans un solvant, dudit premier fragment saccharidique $SM_1$ ;
b) la passivation des groupes fonctionnels hydroxyle de surface n'ayant pas réagi par le contact de la surface du support solide avec au moins un composé de formule (I) ci-dessous :

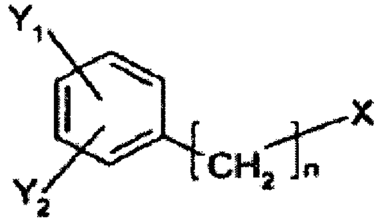

(I)

dans laquelle :

- $Y_1$ et $Y_2$, qui peuvent être identiques ou différents, représentent un atome d'hydrogène, un atome d'halogène, un radical alcoxy en $C_1$ à $C_4$, un radical alkyle linéaire ou ramifié en $C_1$ à $C_4$, un radical thioalkyle en $C_1$ à $C_4$, un groupe nitro, un groupe azido, un radical trifluoroalkyle en $C_1$ à $C_4$, un groupe cyano ou un cycle aryle ;
- n est un nombre entier allant de 1 à 4 inclus ;
- X est choisi dans le groupe constitué d'un atome d'halogène, d'un groupe trichloroacétimide, d'un groupe xanthate $-SC=SOR_1$ où $R_1$ représente un radical alkyle linéaire ou ramifié en $C_1$ à $C_4$, un groupe thioalkyle en $C_1$ à $C_4$, un groupe thioaryle, un groupe phosphate, un groupe phosphite, un groupe séléno-alkyle en $C_1$ à $C_4$, un groupe séléno-aryle, un radical alcoxy en $C_1$ à $C_5$ ou un groupe sulfoxyde $-S(O)-R_2$ dans lequel $R_2$ représente un radical alkyle linéaire ou ramifié en $C_1$ à $C_4$ ou un cycle aryle ;

à condition que quand un parmi $Y_1$ et $Y_2$ représente un hydrogène et que l'autre parmi $Y_1$ et $Y_2$ est un radical méthoxy, le radical méthoxy ne soit pas en position para par rapport à l'atome de carbone portant la chaîne $-(CH_2)_n-X$ ;

c) la répétition de l'étape a) de couplage jusqu'à l'obtention d'une pluralité de molécules de type saccharide ayant des séquences saccharidiques déterminées, chaque étape a) de couplage étant réalisée sur au moins une zone sélectionnée $A_2$, $A_3$, $A_4$..., $A_m$ de la surface, ladite zones $A_2$, $A_3$, $A_4$..., $A_m$ étant identique à ou au moins partiellement différente de la zone de l'étape de couplage précédente, avec un fragment saccharidique $SM_1$, $SM_2$, $SM_3$, $SM_4$..., $SM_m$ possédant au moins une fonction hydroxyle protégée par un groupe protecteur P, ledit fragment saccharidique étant identique à ou différent du fragment saccharidique couplé lors l'étape de couplage précédente, chaque étape a) de couplage étant suivie d'une sous-étape d'élimination du groupe protecteur P d'au moins une fonction hydroxyle du fragment saccharidique couplé lors l'étape de couplage précédente ;

d) la déprotection des fonctions hydroxyle des molécules de type saccharide qui sont encore protégées par un groupe protecteur P.

**2.** Procédé selon la revendication 1, dans lequel il utilise un support qui ne porte pas naturellement des groupes fonctionnels hydroxyle et dans lequel il comprend une étape préliminaire d'hydroxylation de la surface qui est réalisée par la réaction de régions au moins sélectionnées de la surface dudit support avec une solution, dans un solvant organique, d'un espaceur portant au moins un groupe fonctionnel hydroxyle terminal pour obtenir une surface modifiée par des groupes fonctionnels hydroxyle sur lesdites régions au moins sélectionnées.

**3.** Procédé selon la revendication 2, dans lequel l'espaceur portant au moins une fonction hydroxyle terminale est un agent de silylation choisi parmi les composés de formules (II-a) et (II-b) suivantes :

$$M-(CH_2)_x-O-R_6 \qquad \text{(II-a)}$$

et

$$M-(CH_2)_x-R_7 \qquad \text{(II-b)}$$

dans lesquelles :

- M représente un groupe silanisé $-Si(R_3)_3$, $-SiR_3(R_4)_2$ ou $-SiR_3R_4R_5$, où $R_3$, $R_4$ et $R_5$ représentent chacun indépendamment un hydrogène ou un atome d'halogène, tel que des atomes de fluor et de chlore, un radical

alcoxy en $C_1$ à $C_4$, un radical alkyle en $C_1$ à $C_4$ ou un radical chloro-alkyle en $C_1$ à $C_4$ ;
- x est un nombre entier allant de 1 à 20 inclus ;
- $R_6$ représente un groupe protecteur de fonction hydroxyle ;
- $R_7$ représentent un précurseur d'un groupe contenant au moins une fonction hydroxyle, tel qu'un groupe époxyde.

**4.** Procédé selon la revendication 3, dans lequel l'agent de silylation est le 5, 6-époxyhexyltriéthoxysilane ou le trifluorométhoxyundécanetriméthoxysilane.

**5.** Procédé selon l'une quelconque des revendications précédentes, dans lequel le fragment saccharidique est choisi parmi les monosaccharides, les diholosides et les oligosaccharides.

**6.** Procédé selon l'une quelconque des revendications précédentes, dans lequel le solvant utilisé dans les étapes a) et c) est choisi parmi le dichlorométhane, le chloroforme, l'acétonitrile, le diéthyléther et le toluène.

**7.** Procédé selon l'une quelconque des revendications précédentes, dans lequel les composés de formule (I) sont choisis dans le groupe constitué des composés dans lesquels

i) n = 1, un parmi $Y_1$ et $Y_2$ est un atome d'hydrogène et l'autre parmi $Y_1$ et $Y_2$ est un hydrogène ou un atome d'halogène, un radical alcoxy en $C_1$ à $C_4$, un radical thioalkyle en $C_1$ à $C_4$ ou un cyano ou un groupe azido et X est un atome d'halogène ou un groupe trichloroacétimide ou thioalkyle en $C_1$ à $C_4$;
ii) n = 1, $Y_1$ et $Y_2$ sont identiques et représentent un radical alcoxy en $C_1$ à $C_4$ ou un radical alkyle en $C_1$ à $C_4$ et X représente un atome d'halogène ou un groupe trichloroacétimide ou un groupe thioalkyle en $C_1$ à $C_4$ ;
iii) n = 2, un parmi $Y_1$ et $Y_2$ est un atome d'hydrogène et l'autre parmi $Y_1$ et $Y_2$ est un hydrogène ou un atome d'halogène, un radical alcoxy en $C_1$ à $C_4$ ou un cyano ou un groupe azido et X est un groupe trichloroacétimide ;
iv) n = 2, $Y_1$ et $Y_2$ sont identiques et représentent un radical alcoxy en $C_1$ à $C_4$ et X représente un groupe trichloroacétimide.

**8.** Procédé selon l'une quelconque des revendications précédentes, dans lequel les composés de formule (I) sont choisis parmi l'ester de benzyle de l'acide 2, 2, 2-trichloroacétimidique ; le chlorure de benzyle ; le bromure de benzyle ; le bromure de 2-(trifluorométhyl) benzyle ; le bromure de 3, 5-di-(tert-butyl) benzyle et le chlorure de 4-(méthylthio) benzyle.

**9.** Procédé selon l'une quelconque des revendications précédentes, dans lequel la totalité de la surface du support solide est modifiée avec des groupes fonctionnels hydroxyle de surface et dans lequel l'étape a) est précédée par une étape de formation de masque/suppression de masque comprenant les sous-étapes suivantes :

1) le dépôt d'au moins un polymère de protection sur au moins une région sélectionnée de la surface hydroxylée par le microdépôt de gouttes dudit polymère en solution dans un solvant organique pour former des coiffes de polymère solide sur la ou les régions sélectionnées après l'évaporation dudit solvant,
2) la protection des fonctions hydroxyle des régions non coiffées de la surface du support solide avec au moins un composé de formule (I) tel que défini ci-dessus, et
3) l'élimination des coiffes solides de polymère de protection se trouvant précédemment sur la ou les régions sélectionnées par la dissolution des coiffes solides dans un solvant organique.

**10.** Procédé selon l'une quelconque des revendications précédentes, dans lequel les régions sélectionnées de la surface du support solide sont masquées avec au moins un polymère de protection selon l'étape de formation de masque/suppression de masque qui est réalisée avant l'étape a) et/ou avant et entre chaque étape c).

**11.** Procédé selon la revendication 10, dans lequel l'étape de formation de masque/suppression de masque est divisée en au moins deux sous-étapes, une sous-étape de formation de masque réalisée avant l'étape a) et/ou avant et entre chaque étape c), puis une sous-étape de suppression de masque réalisée après l'étape a) et/ou après chaque étape c).

**12.** Procédé selon la revendication 11, dans lequel l'étape de formation de masque/suppression de masque comprend les sous-étapes suivantes :

1) une sous-étape de formation de masque par le dépôt d'au moins un polymère de protection sur au moins

une région sélectionnée de la surface du support solide par le microdépôt de gouttes dudit polymère pour former des coiffes de polymère solide sur la ou les régions sélectionnées après l'évaporation dudit solvant,
2) le couplage d'un premier fragment saccharidique selon l'étape a) décrite dans la revendication 1 et/ou le couplage d'un fragment saccharidique supplémentaire selon l'étape c) décrite dans la revendication 1, et
3) une sous-étape de suppression de masque par l'élimination des coiffes solides de polymère de protection se trouvant sur la ou les régions sélectionnées par la dissolution desdites coiffes solides dans un solvant organique.

**13.** Procédé selon l'une quelconque des revendications 9 à 11, dans lequel le polymère de protection est choisi dans le groupe formé par les polymères de poly(alcool vinylique), de polystyrène, de polyvinylcarbazole, de polyimide et leurs dérivés, ces polymères n'étant ni solubles dans les solvants utilisés pour la réaction lors de l'hydroxylation de la surface du support (avant l'étape a)), ni solubles dans le ou les solvants utilisés lors du couplage des fragments saccharidiques (étapes a) et c)).

**14.** Procédé selon la revendication 13, dans lequel le polymère de protection est choisi parmi les polyhydroxystyrènes qui ne sont pas solubles dans le dichlorométhane.

**15.** Procédé selon l'une quelconque des revendications 9 à 14, dans lequel le solvant organique utilisé dans la sous-étape de suppression de masque pour dissoudre les coiffes de polymère de protection est choisi dans le groupe comprenant le tétrahydrofuranne, l'acétonitrile, l'éthanol, le méthanol, l'acétone et le diméthylsulfoxyde.

**16.** Procédé selon l'une quelconque des revendications précédentes, dans lequel une étape de passivation des fonctions hydroxyle n'ayant pas réagi présentes sur les fragments saccharidiques déjà greffés sur la surface du support solide et qui n'ont pas réagi avec un autre fragment saccharidique lors d'une étape de couplage ultérieure, est réalisée avec au moins un composé de formule (I) tel que défini dans la revendication 1 entre chaque répétition de l'étape c) de couplage d'un fragment saccharidique.

**17.** Procédé selon l'une quelconque des revendications précédentes, dans lequel il comprend, à la fin de la synthèse, une étape supplémentaire d'activation des molécules de type saccharide.

**18.** Procédé selon la revendication 17, dans lequel l'étape d'activation consiste à éliminer les différents groupes protecteurs présents sur les groupes fonctionnels hydroxyle/amine/carboxyle des fragments saccharidiques.

**19.** Support solide comprenant au moins une surface fonctionnalisée avec une ou plusieurs molécules de type saccharide, **caractérisé en ce que** ledit support est obtenu selon le procédé de fabrication défini dans l'une quelconque des revendications précédentes.

**20.** Utilisation d'au moins un support solide tel que défini dans la revendication 19 pour l'identification, par criblage, de molécules de saccharide ou de protéine ou pour l'étude des interactions saccharides/protéines.

**21.** Procédé de criblage de molécules de saccharide ou, respectivement, de ligands protéiques, **caractérisé en ce qu'**il comprend au moins une étape dans laquelle un support solide comprenant au moins une surface fonctionnalisée avec au moins une molécule de type saccharide et préparée selon le procédé défini dans l'une quelconque des revendications 1 à 18 est mis en contact avec une solution contenant une ou plusieurs molécules de saccharide potentielles ou, respectivement, une ou plusieurs protéines potentielles.

**Capping compounds use study**

1/ Polymer masking
2/ Caping compounds coupling
3/ Polymer removing
4/ Mannose glycosylation
5/ Recognition by lectine for mannose

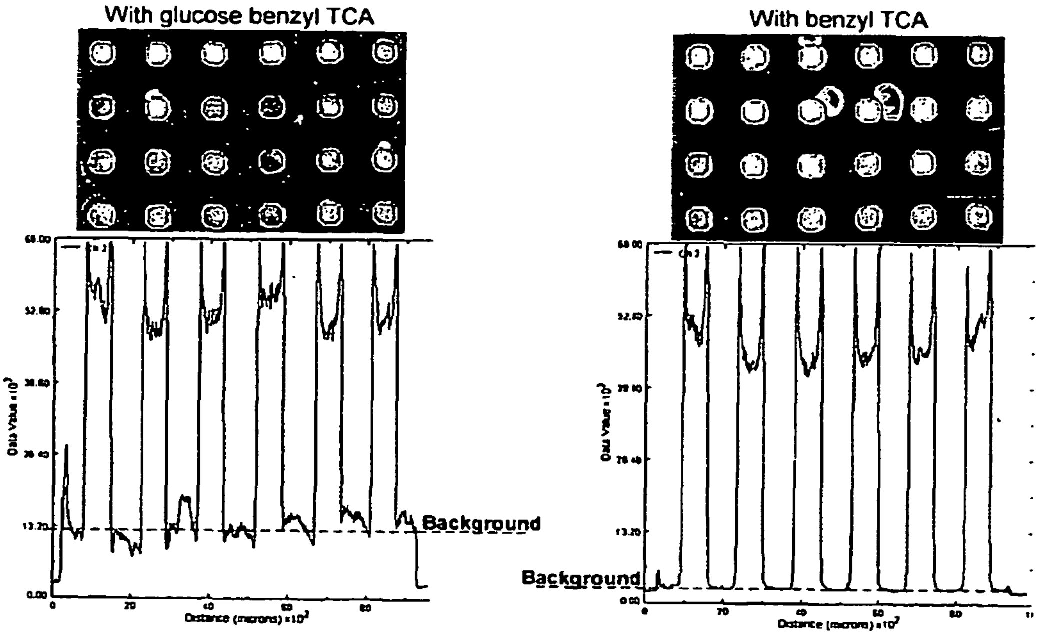


**FIGURE 1**

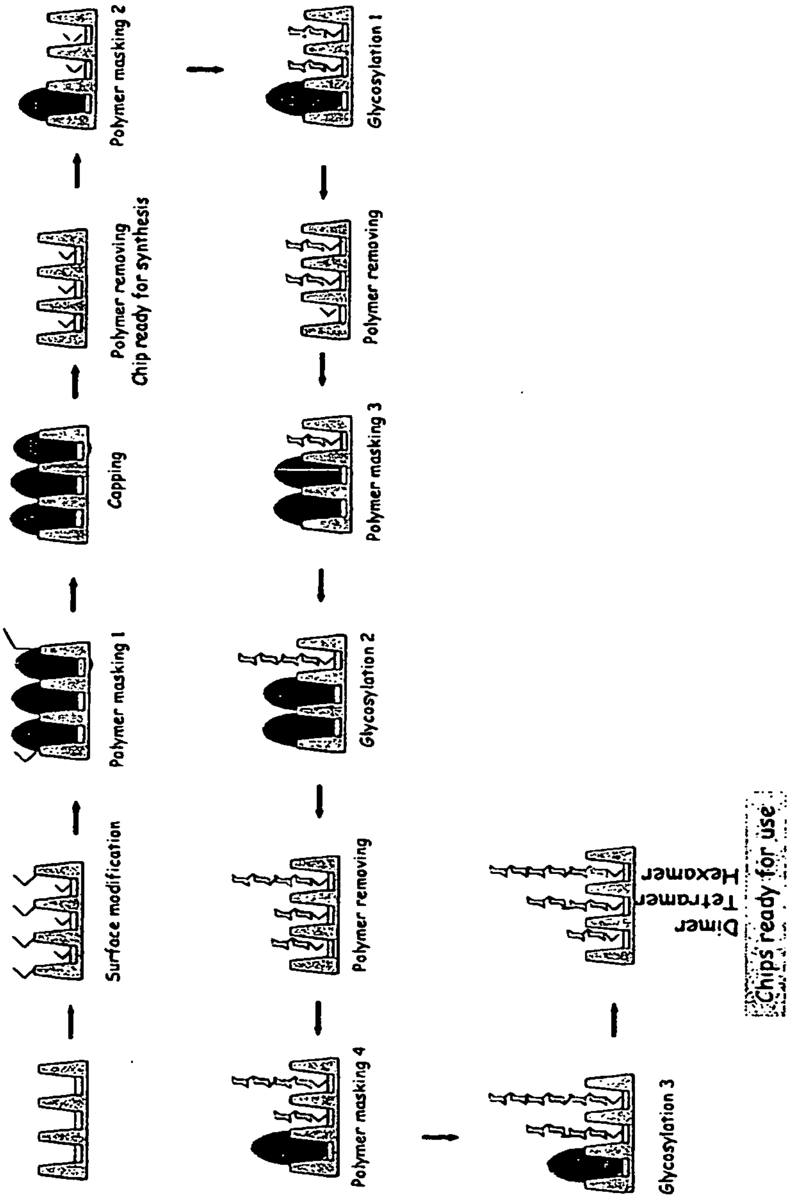
Surface modification
Polymer masking 1
Capping
Polymer removing
Chip ready for synthesis
Polymer masking 2
Glycosylation 1
Polymer removing
Polymer masking 3
Glycosylation 2
Polymer removing
Polymer masking 4
Glycosylation 3
Dimer
Tetramer
Hexamer
Chips ready for use

FIGURE 2

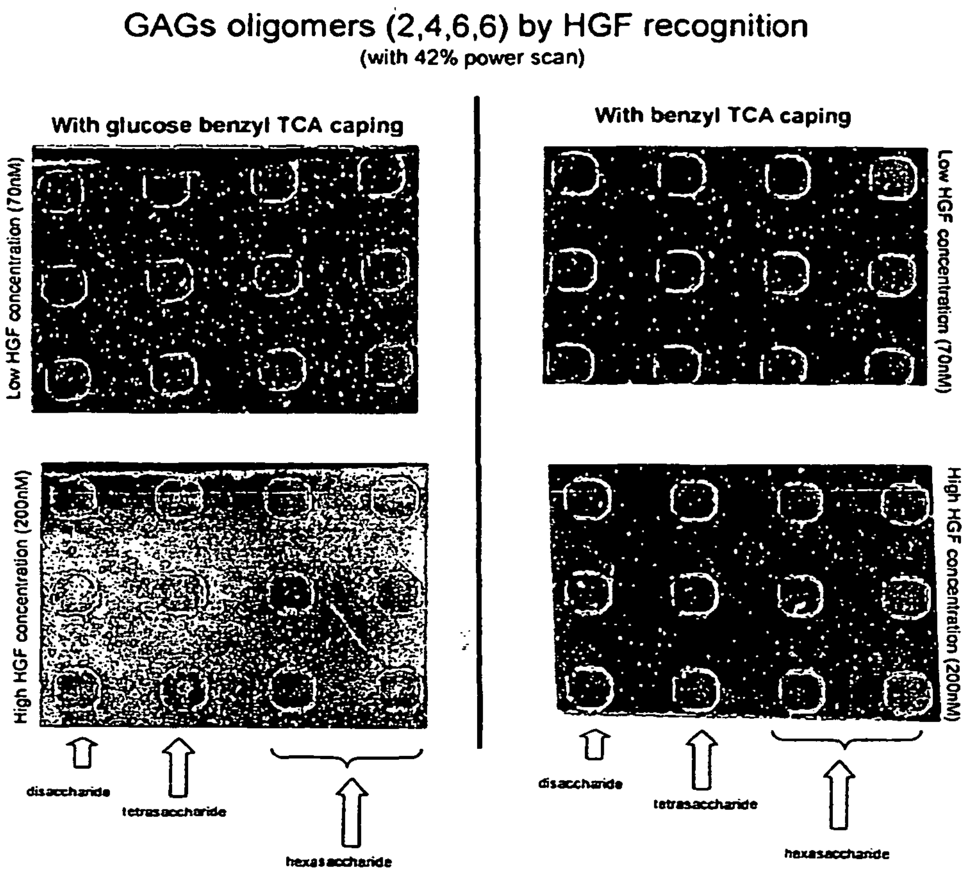
GAGs oligomers (2,4,6,6) by HGF recognition
(with 42% power scan)
With glucose benzyl TCA caping
With benzyl TCA caping
Low HGF concentration (70nM)
High HGF concentration (200nM)
Low HGF concentration (70nM)
High HGF concentration (200nM)
disaccharide
tetrasaccharide
hexasaccharide
disaccharide
tetrasaccharide
hexasaccharide

**FIGURE 3**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*


### Patent documents cited in the description

- US 5474796 A
- US 5658734 A
- WO 9739151 A
- EP 0728520 A
- EP 1163049 A
- WO 03008927 A

### Non-patent literature cited in the description

- **T. W. GREENE et al.** Protective Groups in Organic Chemistry. Wiley-Interscience Publication, 1991
- **THEODORA W. GREEN ; PETER GM WUTS.** Protective groups in organic synthesis. John Wiley & Sons, Inc, 1999
- **BONNAFFÉ et al.** *European Journal of Organic Chemistry,* 2003, 3603-3620
- **A. LUBINEAU et al.** *Chemical European Journal,* 2004, vol. 10, 4265-4282